# EUROPÄISCHE PATENTSCHRIFT

(11) **EP 1 268 743 B1**
(45) Veröffentlichungstag und Bekanntmachung des Hinweises auf die Patenterteilung: **10.09.2003**
(21) Anmeldenummer: 01936169.0
(22) Anmeldetag: 02.04.2001
(51) Int. Cl.: C12M 3/02, C12M 1/16, C12M 1/26

(54) **VORRICHTUNG ZUM KULTIVIEREN VON PFLANZLICHEN ODER TIERISCHEN GEWEBEKULTUREN**
DEVICE FOR CULTIVATING PLANT OR ANIMAL TISSUE CULTURES
DISPOSITIF DE CULTURE DE TISSUS VEGETAUX OU ANIMAUX

(30) Priorität: 03.04.2000 DE 10016554
(43) Veröffentlichungstag der Anmeldung: 02.01.2003
(73) Patentinhaber: Rootec Gesellschaft für Bioaktive Wirkstoffe MBH, 69123 Heidelberg (DE)
(72) Erfinder: WILDI, Eckhart, 74927 Eschelbronn (DE); WILDI, Reinhart, CH-4102 Binningen (CH); RIPPLINGER, Peter, 69151 Neckargemünd (DE)
(74) Vertreter: Patentanwälte Zellentin & Partner
(86) Internationale Anmeldenummer: EP0103716
(87) Internationale Veröffentlichungsnummer: WO01074992

(56) Entgegenhaltungen:
- CH-A- 372 911
- FR-A- 2 786 783
- US-A- 5 350 080

## Beschreibung

Gegenstand der vorliegenden Anmeldung ist eine neue Vorrichtung zum Kultivieren von pflanzlichen oder tierischen Zell-, Gewebe- oder Organkulturen (im folgenden Gewebe genannt), sowie Kultivierungsverfahren unter Verwendung solcher Vorrichtungen.

Die Kultivierung von Zellen zur Herstellung von Zellmaterial und insbesondere von metabolischen Produkten dieser Zellen gewinnt ständig an Bedeutung, da die chemische Synthese solcher Produkte häufig schwierig oder unmöglich ist oder sich gegenüber der biochemischen Produktion als unwirtschaftlich erweist. Neben der bereits seit langem im großen Umfang ausgeführten Kultivierung von Hefen, Schimmelpilzen und Bakterien gewinnt für die Herstellung bestimmter Produkte in zunehmendem Maße auch die Kultivierung von Pflanzen- oder Tierzellen an Bedeutung. Die Erforschung geeigneter Kultivierungsbedingungen und gegebenenfalls die genetische Veränderung und Züchtung von in der Natur vorkommenden Pflanzen- oder Tierzellen zur Erzielung von hohen Ausbeuten sind dabei vordringliche Ziele der Forschung.

Man unterscheidet pflanzliche oder tierische Zell- und Gewebekulturen (vorwiegend Zellsuspensionen, Kalluskulturen) von pflanzlichen Organkulturen (transformierten Wurzelhaar- (Hairy root), Wurzel- oder Sprosskulturen (shooty teratomas)). Für die Fermentation wurden bislang vor allem Zellsuspensionskulturen eingesetzt, welche als undifferenzierte Einzelzellen oder als Zellaggregate bestehen. Seit den achtziger Jahren waren intensive Anstrengungen im Gange, diese Zellsuspensionskulturen in Large-Scale-Fermentern zu kultivieren und für die kommerzielle Produktion von Gewebeinhaltsstoffen zu nutzen.

Von einigen Ausnahmen abgesehen, hat sich aber gezeigt, daß Zellsuspensionskulturen genetisch häufig instabil sind (somaklonale Variation). Dies hat einen gravierenden Einfluß auf die Wirkstoffproduktion, da selbst vielversprechende Hochleistungszelllinien nach einigen Zyklen starken Schwankungen unterworfen sein können oder die Produktion der Wirkstoffe vollständig unterbunden wird. Man hat diese Instabilität dem Einfluß der Wachstumsregulatoren, sowie dem undifferenzierten Zustand der Zellen zugeschrieben, welche häufig ohne ausreichende Ausdifferenzierung von Zellorganellen, oder ohne entsprechenden Zell-Zellkontakt einzelne Biosynthesewege nicht stabil ausprägen. Epigenetische Faktoren spielen somit für die Biosynthese einen ganz wesentlichen Einfluß.

Pflanzliche Organkulturen und hier vor allem die kommerziell interessanten transformierten Wurzelhaarkulturen bilden ausdifferenziertes Gewebe, welches sich auch in Langzeitkulturen als genetisch deutlich stabiler erweist als Zellsuspensionskulturen. Dank des schnellen Wachstums von transformierten Wurzelhaarkulturen, weiche häufig vergleichbare Wachstumsraten wie Zellsuspensionskulturen aufweisen, sind diese Organkulturen für die fermentative Produktion von kommerziell interessanten Inhattsstoffen geeignet. Vor allem können transformierte Wurzelhaarkulturen - anders als Zellsuspensionskulturen - ohne Wachstumsregulatoren kultiviert werden. Beispielhaft sei auf die Synthese von Taxoiden, Podophyllotoxinen oder Rosmarinsäuren verwiesen, die nach solchen Verfahren hergestellt wurden. Einzelne Wachstumsregulatoren können die Biosynthese von Sekundärmetaboliten hemmen.

Allerdings benötigen pflanzliche oder tierische Organkulturen ein völlig neues Fermenterdesign, da ein Scale-up der Laboranlagen, bedingt durch das inhomogene und nicht zu durchmischende Gewebe, die Gas- und Nährlösungsversorgung erschwert.

Zur Züchtung von Geweben ist es notwendig, die für die Ernährung der Zellen notwendigen Mineralstoffe, Wachstumsregulatoren, Kohlenstoffquellen, normalerweise Saccharose, Fructose oder Glucose, sowie gegebenenfalls Gase wie Sauerstoff oder Kohlendioxid den Zellen regelmäßig zuzuführen.

Die einfachste und wirtschaftlichste Form der Kultivierung von Zellen ist die Suspensionszellkultur, wobei isolierte Zellen in einer Nährflüssigkeit suspendiert sind, verbrauchte Nahrungsbestandteile der Nährlösung regelmäßig zugeführt werden und gegebenenfalls eine Begasung zur Aufrechterhaltung der Suspension und Ernährung der Zellen durchgeführt wird. Durch entsprechende Wachstumsregulatorien wird ein Zusammenwachsen der Zellen zu größeren Aggregaten verhindert. Als nachteilig erweist sich dabei, daß viele Pflanzen- oder Tierzellen in dieser Form nicht lange lebensfähig sind und die Bildung von aus der Kulturflüssigkeit schlecht eliminierbaren Metaboliten eine häufige Übertragung der Zellen in frische Nährlösung erfordert.

Für kleinere Kulturen ist es möglich, die Zellen auf der Oberfläche von nährstoffhaltigen Gelen, beispielsweise auf mit Agar ausgegossenen Petrischalen zu impfen, wobei die Zellen aus dem Gel und der umgebenden Atmosphäre die notwendigen Nahrungsbestandteile zu sich zu nehmen. Auch bei dieser Methode erweist es sich als nachteilig, daß viele Zellen unter diesen Bedingungen schlecht und nur langsam wachsen und Metaboliten abscheiden, die sich teilweise im umgebenden Medium anreichern und wiederum das Wachstum der Zellen und die Zellteilung verlangsamen oder die Zelle selbst töten. Durch die Fixierung auf der Geloberfläche wachsen die Zellen zu größeren Haufen aus, was wiederum den Nachteil hat, daß nur die Oberfläche mit Gas und die Unterseite mit der Nährflüssigkeit in Kontakt ist und somit die Nahrungszufuhr sich beim Wachstum verlangsamt. Auch hier ist, um ein ausreichendes Wachstums aufrecht zu erhalten, eine häufige Überimpfung der Kulturen auf neue Nährboden erforderlich.

Um die Nachteile der Suspensionskultur isolierter Zellen zu vermeiden, ist man daher dazu übergegangen, ausdifferenzierte Kulturen wie "Hairy roots" oder Pflanzensprossen bzw. Blattgewebe zu kultivieren. Solche größeren Aggregate neigen in Suspension zur Enthomogenisierung, insbesondere in größeren Reaktoren, wodurch eine Abänderung bzw. Anpassung der Verfahrensbedingungen erforderlich wurde, durch die eine gleichmäßige Versorgung der Zellaggregate mit Nährlösung und den notwendigen Gasen erreicht wird.

Ein verbreitetes Verfahren beruht darauf, die Gewebe auf festen Trägern zu fixieren und eine dünne Schicht von Nährflüssigkeit über die Träger laufen zu lassen, so daß die Gewebe laufend mit frischer Nährflüssigkeit versorgt werden und gleichzeitig auch einen ausreichenden Kontakt zur Gasatmosphäre im Reaktor besitzen. Als Trägerkörper werden Platten oder Gewebe, insbesondere Maschengitter oder Stabkonstruktionen als Unterlage verwendet, die in entsprechenden Reaktoren parallel im Abstand zueinander angeordnet sind, so daß die Zwischenräume eine Beaufschlagung mit Nährflüssigkeit und Gas und ein gewisses Wachstum der Zellkultur ermöglichen. Die Fixierung der Zellen auf dem Träger erfolgt dabei z.B. durch Einklemmen in Spalten oder Winkel der Trägeroberfläche (vgl. EP 234 868 und US 5,585,266). Für die Fermentation von sogenannten "Hairy root-Kulturen" wird die Verwendung von Fermentern, welche ein System von gespannten Drähten enthalten, besonders empfohlen, wobei der Abstand der Drähte an den Kreuzungspunkten so klein ist, daßan diesen Stellen das Pflanzengewebe festgehalten wird oder durch spezielle Ausbildung der Drähte mit Stacheln in diesen Stachelachseln festgehalten werden (vgl. WO 89/10958).

Die Fermentationsgefäße des Standes der Technik bestehen aus Stabilitätsgründen üblicherweise aus Metall, beispielsweise Stahl- oder Aluminiumblech oder wegen der Durchsichtigkeit aus Glas oder Acrylglas und haben überwiegend eine kubische Form, welche eine platzsparende Anordnung mehrerer paralleler Trägerplattensysteme im Inneren ermöglicht. Ein abnehmbarer Deckel, an dem gegebenenfalls neben den Zuführungsleitungen auch die Trägerplatten befestigt sind, erlaubt einen Zugang zu dem Behälter, insbesondere zum Abernten der Zellkultur und zum Reinigen. Die Herstellung der Fermentationsgefäße aus stabilen Materialien erlaubt, solche Reaktoren standfest nebeneinander auf dem Boden anzuordnen, hat jedoch den Nachteil, daß die Herstellung und Wartung solcher Gefäße teuer ist und eine Adaption an die Kulturgröße nicht erlaubt.

Es stellte sich daher die Aufgabe, einfache und wirtschaftliche Vorrichtungen zur Kultivierung von Gewebe, insbesondere von Pflanzengewebe zu finden, welche einfach und preiswert herzustellen, in der Größe veränderbar sind und eine einfache Handhabung und Wartung erlauben.

Diese Aufgabe wird durch die im Hauptanspruch wiedergegebene Vorrichtung gelöst und durch die Merkmale der Unteransprüche gefördert.

Insbesondere ist es Aufgabe der vorliegenden Erfindung, unter Verwendung solcher Fermentationsvorrichtungen ein Verfahren zur Kultivierung von Pflanzengewebe des "Hairy root-Typs oder Pflanzensproß-Typs" bereitzustellen.

Überraschenderweise war es möglich, die starren Wände der Fermentationsvorrichtungen des Standes der Technik durch topf- oder beutelförmige Plastikschläuche aus dünner Kunststofffolie zu ersetzen, wenn man diese über einfache, bekannte Verbindungsvorrichtungen, insbesondere Quetschverbindungen, direkt an dem die Nährmittel und Gas zu- und abführenden Leitungen tragenden Deckel eines üblichen Fermentationsgefäßes befestigt; welcher ebenfalls noch die Trägerplatten für die Fixierung der zu kultivierenden Zellen trägt.

Über am Deckel befestigte Haltevorrichtungen lassen sich solche Vorrichtungen problemlos in Gruppen an entsprechenden Gestellen aufhängen, wobei diese Aufhängegestelle gleichzeitig auch die Zu- und Abführungsleitungen für die Nährlösungen und Gase tragen, die von zentralen Versorgungs- und Steuereinheiten in ihrer Zusammensetzung reguliert werden können. Durch ein modulares System der Zu- und Abführungsleitungen innerhalb der Fermentationsvorrichtung aus kurzen Rohrstücken mit zwischengesetzten Verbindungsstücken und einer Anordnung der Trägerplatten an diesen Rohrabschnitten lassen sich in der Länge und damit in der Kapazität veränderbare Fermenter leicht zusammensetzen und mit Schlauchfolien entsprechender Länge als Außenwand versehen, so daß es möglich ist, die Kapazität eines solchen Fermenters in bestimmten Stufen zu verändem. Die Schlauchfolie ist üblicherweise unten mit einem eingesetzten Plastikboden oder durch Zusammenquetschen und Verschweißen verschlossen, jedoch ist es auch möglich, einen dem Deckel im Durchmesser entsprechenden Bodeneinsatz aus festem Werkstoff mit einer entsprechenden Quetschdichtung einzufügen, in dem gegebenenfalls weitere Zu- und Abführungsleitungen befestigt sein können. Die Herstellung des Fermenterbehälters aus lichtdurchlässiger Plastikfolie erlaubt weiterhin, die im Inneren stattfindenden Reaktionen von außen zu beobachten sowie gegebenenfalls durch die Wand Licht- oder Wärmestrahlung, welche insbesondere Pflanzengewebe zur Kultur gegebenenfalls benötigen, zuzuführen. Einen besonderen Vorteil stellt es dar, daß zur Entnahme der kultivierten Gewebe für die Herstellung von Subkulturen oder die Aufarbeitung auf eventuelle Zellinhaltsstoffe der äußere Plastikschlauch aufgeschnitten oder auch nach Lösen der Klemmverbindung der Deckel ohne Lösung der Zuführungsleitungen zum Deckel mit den daran befestigten Trägerplatten gegebenenfalls unter einem sterilen laminaren Schutzgasstrom, der hochgezogen werden kann, so daß die Kulturträgerplatten frei liegen und für eine einfache Bearbeitung zur Verfügung stehen. Die bei konventionellen Behältern aus festem Material erforderliche Reinigung des Behälters entfällt, da die Plastikschläuche nach Benutzung verworfen und durch neue in geeigneter Größe ersetzt werden.

Die Plastikschläuche können aus allen handelsüblichen Folien bestehen, Polyethylen, Polypropylen, Polyvinylchlorid oder Polyesterfolien sind besonders bevorzugt, da sie nicht nur besonders wirtschaftlich herzustellen, sondern auch in ihrer chemischen Beständigkeit, Elastizität, Lichtdurchlässigkeit und Festigkeit allen notwendigen Anforderungen entsprechen. Bei der Auswahl der Plastikfolie muß lediglich darauf geachtet werden, daß diese aus der Herstellung keine Weichmacher oder sonstigen Hilfsstoffe enthält, welche gegebenenfalls als Giftstoffe für die Fermentation wirken können, wenn sie aus der Folie durch die Nährlösung herausgelöst werden.

Als Träger für die zu kultivierenden Gewebe dienen vorzugsweise Gitter oder parallel angeordnete Stäbe aus biologisch inerten Materialien, z.B. rostfreiem Stahl, Kunststoff oder einem in einem Rahmen eingespannten Textilgewebe, welche als radiale Platten von dem zentralen Zuführungsrohr der Nährlösung abstehen, wobei diese Zuführung vorzugsweise aus mehreren kurzen Rohrstücken besteht, an denen entsprechend breite Platten befestigt sind, und die Rohrstücke durch Zwischenstücke verbunden sind, die als Austrittsdüsen für die Nährflüssigkeit dienen, so daß Nährflüssigkeit in regelmäßigen Abständen in den Fermentationsbehälter eingedüst werden kann und somit eine gleichmäßige Befeuchtung der Kulturflächen von der Mitte aus bewirkt Alternativ kann auch über separate Zuführungsleitungen im äußeren Bereich zwischen den radialen Platten eine Eindüsung der Nährlösung erfolgen, wobei das zentrale Rohr dann nur als Träger der Platten fungiert.

Durch Einspannen entsprechend dimensionierter Gitter aus Stahldraht oder Kunststoff in einen festen äußeren Rahmen, der vorzugsweise aus rostfreiem Stahl besteht, lassen sich die Trägersysteme einerseits den zu kultivierenden Pflanzengeweben in der Größe und Ausbildung der Kontaktstellen (Kreuzungspunkte) anpassen, andererseits zur Aberntung der Zellgewebe und zur Reinigung auseinandernehmen.

Alternativ zu einer radialen Anordnung ist es möglich, die Platten auch quer zur Richtung der zentralen Führung anzuordnen, wobei ein 0-90° Winkel zwischen Platte und Führung es erlaubt, die Abflußgeschwindigkeit der Nährflüssigkeit über die Platte zu beeinflussen, die um so höher ist, je steiler die Platte im Reaktor steht. Vorzugsweise läßt sich dieser Winkel deshalb über entsprechende Kippvorrichtungen, beispielsweise Scharniere, einstellen. Lagerung und Reinigung der Vorrichtung wird durch diese Kippmöglichkeit ebenfalls erleichtert.

Die Ausbringung der Nährflüssigkeit in Form feiner Nebeltröpfchen ist an sich bekannt (EP 0 234 868 B1) und hat sich für die Nahrungszufuhr für Pflanzenzellen besonders bewährt, da gleichzeitig auch ein hoher Gasaustausch bewirkt wird. Überschüssige oder verbrauchte Nährlösung kann aufgrund der Schwerkraft von den Platten zum Boden des Gefäßes abtropfen, von wo durch eine entsprechende Absaugvorrichtung die Flüssigkeit nach Regeneration, d.h. der Versorgung mit den verbrauchten Nährstoffen und der Abtrennung der gebildeten Metabolite, wieder in den Fermenter zurückgeführt werden kann. Auch die Absaugrohre werden in entsprechenden modularen Einheiten vorgesehen, wobei die Zwischenstücke natürlich nicht als Düsen, sondern als durchgehende Verbindungen ausgebildet sind, um auch insoweit eine Größenänderung der Fermenter zu ermöglichen.

Die Zuführung der Nahrlösung erfolgt über übliche 1- oder 2-Stoffdüsen. Vorzugsweise wird jedoch ein aus einer Bohrung in der Nährlösungszuleitung austretender Flüssigkeitsstrahl über Gasstrahlen, die aus benachbarten Gasleitungen austreten und einen Gasaustausch im System bewirken, verwirbelt und in feine Tröpfchen zerlegt. Bohrungsgröße, Druck von Flüssigkeit und Gas und die Geometrie der zusammentreffenden Strahlen lassen die Vernebelung in weiten Grenzen steuern.

Im einfachsten Fall sind bei der erfindungsgemäßen Vorrichtung alle Elemente wie Trägerplatten, Nährflüssigkeitszu- und -ablaufvorrichtungen und Gasleitungen zentral am Deckel befestigt. Um das Abernten der Gewebe bzw. einen Kulturaustausch zu erleichtern, kann es jedoch vorteilhaft sein, den Deckelteil in einem äußeren Ring, der die Versorgungsleitungen trägt und einen mittleren Deckelteil an dem das Trägerplattensystem befestigt ist, zu trennen. Durch Hebevorrichtungen kann so das Trägerplattensystem aus dem Fermenter herausgezogen werden, ohne die Anschlüsse der Versorgungsleitungen zu lösen.

Vorzugsweise werden bei einer solchen Unterteilung alle Leitungen an einer Seite des äußeren Ringes angebracht und durch Ringleitungen im Umfang des äußeren Ringes weitergeleitet. Dadurch ist es erleichtert, von der anderen Seite in den geöffneten Reaktor einzugreifen und durch Überleiten eines laminaren, sterilen Luftstroms während des Eingreifens eine Kontamination zu verhindern.

Die erfindungsgemäßen Fermenter haben üblicherweise ein Volumen von 10 - 250, vorzugsweise 50 - 100 I und sind mit vier im Kreuz stehenden Trägerplatten ausgerüstet.
Ein 100 I-Fermenter hat beispielsweise ein Höhe von 85 cm bei einem Durchmesser von 28 cm und enthält zwei übereinander angeordnete Plattenkreuze mit 40 cm Höhe und 20 cm Breite.

Soweit horizontale bzw. schräg stehende Plattensysteme verwendet werden, ist deren Abstand mit 5-25 cm, vorzugsweise etwa 10 cm, zu wählen. Bei dem vorstehenden Reaktor können so 4-10 Platten eingesetzt werden.

Diese Anordnung macht es zusätzlich möglich, zwei unterschiedliche Kulturen übereinander anzuordnen, z.B. eine Wurzelzellenkultur im oberen Bereich und eine Blattsproßkultur im unteren Bereich, welche dann gegebenenfalls zusätzlich belichtet werden kann. So können im Wurzelzellbereich stärker gebildete Zwischenprodukte mit der Nährflüssigkeit auf die Blattsproßkultur gebracht werden, wo sie zu den gewünschten Endprodukten weiter umgesetzt werden. Durch die vertikale Anbringung der Träger übereinander tropft das Nährmedium passiv ab und erübrigt einen zusätzlichen Transportaufwand, wie er bisher üblich war, wenn die unterschiedlichen Kulturen in getrennten Fermentern kultiviert wurden.

Die Nährlösung wird kontinuierlich zwischen Fermenter und einem Nährlösungstank zirkuliert. Zwischengeschaltete Absorptionssäulen, in denen die Metabolite der Fermentation an geeignetes Säulenmaterial adsorbiert werden, Filter für aus dem Fermenter ausgeschwemmtes festes Material, Zuführungen für neues Nährmaterial oder Vorrichtungen zum Ausschleusen verbrauchter Lösung sind wie bei bekannten Fermentem vorgesehen.

In einer besonders einfachen und preiswerten Ausführungsform besteht auch der Nährlösungstank aus einem einfachen Plastiksack, der aus Stabilitätsgründen in einer Metall- oder Kunststoffwanne liegt und über Ventilanschlüsse, beispielsweise Luer-Lock-Verschlüsse mit den Versorgungsleitungen verbunden ist.
Eine Magnetrührvorrichtung sorgt für eine kontaminationsfreie Durchmischung des Sackinhalts. Diese preiswerten Tanks können beim Kulturwechsel direkt entsorgt werden und ersparen so die übliche aufwendige Reinigung. Durch Zuordnung jeweils eines oder mehrerer Fermenter zu einem Nährtank in einem fahrbaren Gestell gelangt man zu kompakten, mobilen Einheiten.

In den folgenden Figuren wird die Erfindung näher erläutert, ohne sie jedoch darauf zu beschränken.

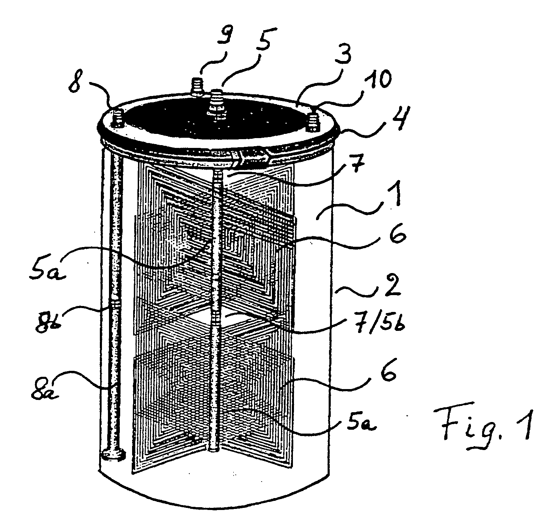

**Fig. 1** zeigt einen Fermenter 1 mit äußerem Plastikschlauch 2 und Deckelteil 3, welcher über einen Quetschverschluß 4 den Schlauch 2 festhält. Der Deckel 3 hält das zentrale Zuführungsrohr 5, an dem die Trägerplatten 6 kreuzförmig befestigt sind, im Zuführungsrohr 5 mit den Rohrabschnitten 5a und Zwischenadaptern 5b sind Sprühdüsen 7 für die Nährlösung integriert. In der Peripherie des Deckels 3 sind ein Absaugrohr 8 für verbrauchte Nährlösung und Zu- und Abführungsanschlüsse 9/10 für Gase vorgesehen. Im dargestellten Fall sind zwei Plattenmodule 6 übereinander vorgesehen.

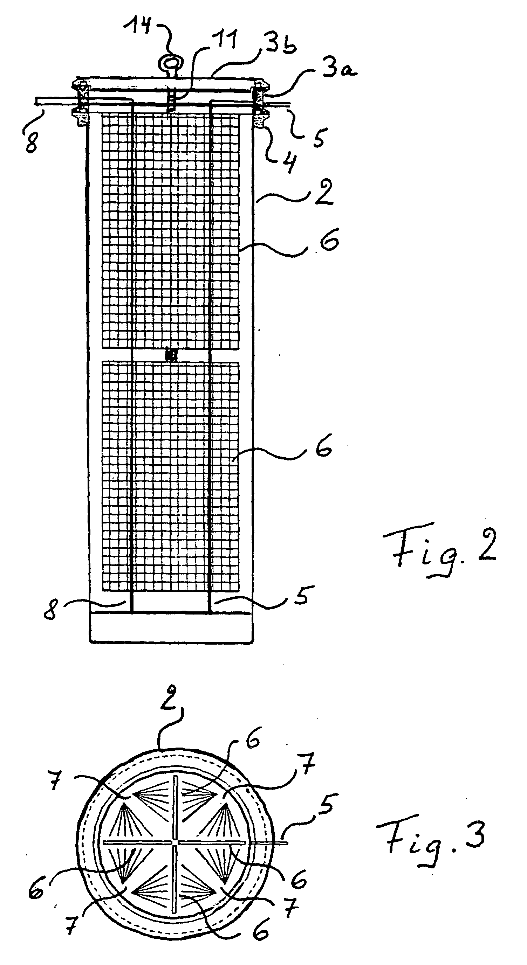

**Fig. 2** zeigt einen Vertikalschnitt durch eine andere Variante, bei der die Zu- und Abführungsleitungen 5, 8, 9, 10 in einem äußeren Deckelring 3a befestigt sind und in der Peripherie des Fermenters 1 nach unten verlaufen. An dem mittleren Deckelaufsatz 3b sind über eine zentrale Halterung 11 die Trägerplatten 6 befestigt. Der Schlauch 2 wird zwischen Deckelring 3a und Spannverbindung 4 festgehalten.

**Fig. 3** zeigt die Zuführung der Nährlösung in einen horizontalen Schnittbild gemäß Fig. 2 über die Sprühdüsen 7, die in der Ringleitung 5c zusammengefaßt sind. Plastikschlauch 2 und Trägerplatten 6 entsprechen Fig. 2.

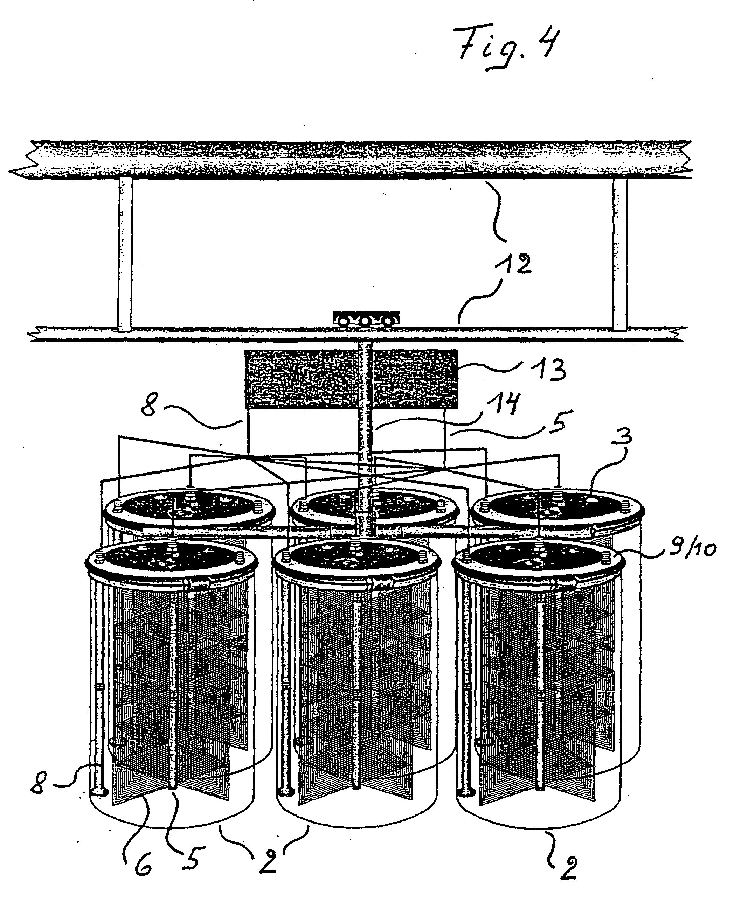

**Fig. 4** zeigt eine Gruppe von Fermentem 1 gemäß Fig. 1, die über eine gemeinsame Haltevorrichtung 14 an einem Schienensystem 12 fahrbar befestigt sind. Nährlösungszuführungsvorrichtungen 5 und -abführungsvorrichtungen 8 sind zusammengefaßt und über eine nicht dargestellte Pumpvorrichtung an den zentralen Nährlösungstank 13 angeschlossen.

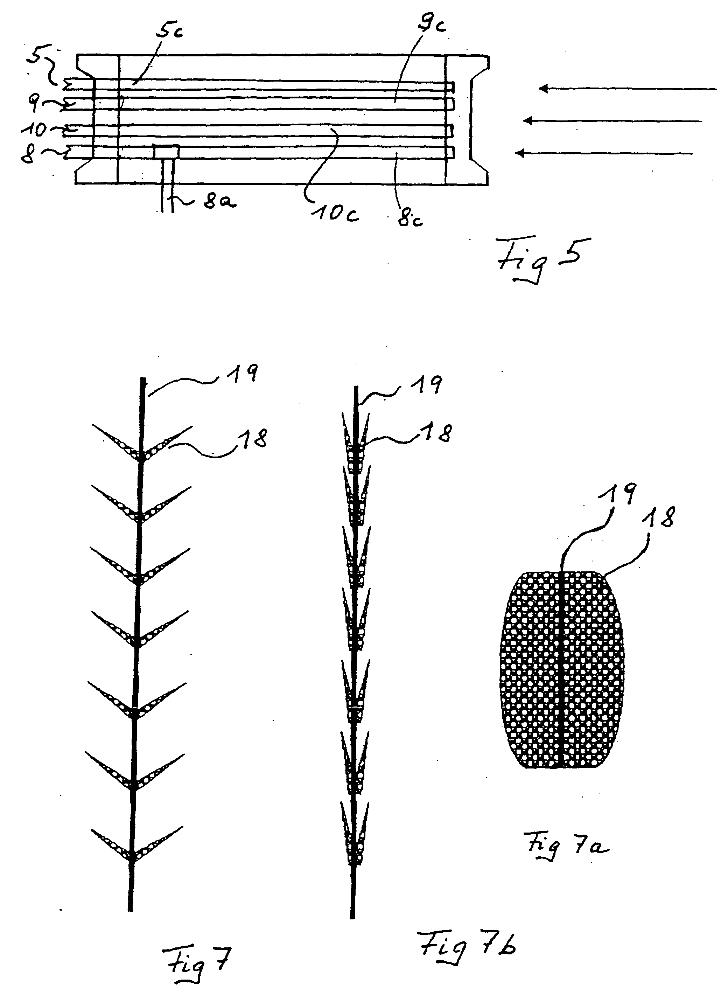

**Fig. 5** zeigt eine bevorzugte Ausbildung des Deckelrings 3a gemäß Fig. 2, bei dem alle Zu- und Abführungsleitungen für Flüssigkeiten und Gase 5, 8, 9 und 10 auf einer Seite angebracht sind und über Ringleitungen 5c, 8c, 9c, 10c eine Verteilung über den Umfang des Reaktors erfolgt und von denen die Weiterleitungen in den Reaktor abgehen. Diese Ausführung hat den Vorteil, daß beim Öffnen des Reaktors von der den Leitungen abgewandten Seite sterile Luft aufgeblasen werden kann, die den Reaktorinhalt vor dem Verkeimen schützt (durch die Pfeile auf der rechten Seite angedeutet). Die Ringleitungen 5c, 8c, 9c, 10c bestehen vorzugsweise aus einem Kunststoffschlauch, der in einer Ausnehmung oder Nut des Deckelrings 3a geführt ist und T-förmige Anschlußstücke für die Leitungen in den Reaktor besitzt. Aus zeichnerischen Gründen ist nur ein Anschluß 8a eingezeichnet.

**Fig. 6** zeigt eine bevorzugte Ausbildung der Sprühdüsen 7 für die Nährlösung, bei der zwei Gaszuführungsrohre 8b durch Haltevorrichtungen 15 parallel mit der Nährlösungszuführung 5b geführt sind. In Höhe der Trägerplatten 6 sind alle drei Rohre 5a, 8a mit 0,1-1 mm großen Bohrungen 7 versehen, so daß der im Betrieb aus dem mittleren Rohr 5a unter Druck austretende Nährlösungsstrahl durch den aus den Gaszuführungsrohen 8a austretenden Gasstrom verwirbelt und in feine Tröpfchen aufgeteilt wird. Bohrungsdurchmesser und Druck, sowie der Winkel unter dem die Gasstrahlen auf die Flüssigkeit auftreffen, erlauben das Sprühbild optimal einzustellen.

**Fig. 6a** zeigt die Anordnung der Sprühdüsenbohrungen 7.
**Fig. 6b** zeigt eine Halterung 15 für die 3 Rohre 5a, 8a, 8a sowie Stellvorrichtungen 16, 17 mit denen die Bohrungen 7 ausgerichtet werden können.

**Fig. 7** zeigt eine weitere Ausbildung der Trägerplatte 6 in Form von gelochten Stahl- oder Aluminiumblechen oder Kunststoffplatten 18, die beidseitig über nicht dargestellte Kippscharniere an einer zentralen Halterung 19 angebracht sind. Die Kippschamiere erlauben es, den Neigungswinkel der Platten 18 (0-90°) und damit die Ablaufgeschwindigkeit der Nährlösung über die Platten einzustellen. Weiterhin ist es möglich die Platten 18 ganz an die Halterung anzulegen, wodurch die Vorrichtung weniger Platz einnimmt, was im unbenutzten Zustand Lagerraum spart und beim Reinigen und Sterilisieren der gebrauchten Vorrichtung kleinere Reinigungsbehälter erfordert (vgl. Fig. 7b).

**Fig. 7a** zeigt schematisch eine Lochplatte 18, sowie im Schnitt die zentrale Halterung 19, die vorzugsweise ebenfalls als gelochte Platte oder Gitterplatte ausgebildet ist, um einen Gas- und Flüssigkeitsdurchtritt zu ermöglichen. Die Platten 18 sind in Abständen von 5-25 cm, vorzugsweise 10 cm, übereinander angeordnet, so daß sich je nach Reaktorhöhe eine Zahl von 4-10 ergibt.

### Bezugszeichenliste

- 1: Fermentergefäß
- 2: Plastikschlauch
- 3: Deckel
- 3a: Äußerer Deckelring
- 3b: Mittlerer Deckelaufsatz
- 4: Spannverbindungen (Quetschverschluß)
- 5: Zuführungsrohr
- 5a: Rohrstücke
- 5b: Zwischenadapter
- 5c: Ringleitung
- 6: Trägerplatten
- 7: Sprühdüsen
- 8: Absaugrohr
- 8a: Rohrstücke
- 8b: Zwischenadapter
- 8c: Ringleitung
- 9/10: Gas-Zu- und Abführungsanschlüsse
- 9c/10c: Ringleitung
- 11: Zentrale Halterung
- 12: Schienensystem
- 13: Nährlösungstank
- 14, 15: Haltevorrichtung
- 16, 17: Stellvorrichtung
- 18: gelochte Platten
- 19: zentrale Halterung

## Patentansprüche

1. Vorrichtung zum Kultivieren von pflanzlichem oder tierischem Gewebe, umfassend ein Fermentergefäß (1), Zuführung für flüssige Nährstoffe und Gase (5) sowie Vorrichtungen zum Abführen von verbrauchten Nährflüssigkeiten und Gasen (8) und festen Trägerplatten (6) für die Gewebe, die für die genannten flüssigen Nährstoffe und die Gase durchlässig sind und innerhalb des Fermentergefäßes (1) ortsfest angeordnet sind, **dadurch gekennzeichnet, daß** das Fermentergefäß (1) aus einem formstabilen Deckelteil (3), welcher die verschiedenen Zu- und Ableitungen (5, 8, 9, 10) sowie die Trägerplatten (6) für die Zellen trägt und aus einem topf- oder beutelförmigen Plastikschlauch (2) besteht, dessen Öffnung gegen den Deckel (3) abgedichtet und befestigt ist.

2. Vorrichtung nach Anspruch 1, **dadurch gekennzeichnet, daß** 3 - 8 Trägerplatten (6) radial an einet zentralen Halterung (11) befestigt sind und die Zuführung der Nährlösung über 3 - 8 im äußeren Bereich zwischen den Platten (6) befindliche Zuführungsleitungen (5) erfolgt.

3. Vorrichtung nach Anspruch 1, **dadurch gekennzeichnet, daß** die Zuführungsleitungen (5a) als Düsen Bohrungen (7) zum Austritt der Nährlösung enthalten und Gaszuführungsleitungen (9) parallel zu der Leitung (5a) geführt sind, welche in entsprechenden Stellen ebenfalls Bohrungen (7) enthalten, aus denen Gas austritt.

4. Vorrichtung gemäß einem der Ansprüche 1 - 3, **dadurch gekennzeichnet, daß** im Außenbereich zwischen den Trägerplatten (6) ein oder mehrere Absaugrohre (8/10) für verbrauchte Nährflüssigkeit und verbrauchte Gase angeordnet sind, welche eine Absaugdüse am Ende enthalten.

5. Vorrichtung gemäß Anspruch 1, **dadurch gekennzeichnet, daß** die Zuführungsleitung ein zentrales Rohr (5) darstellt, welches aus zwei oder mehreren über Zwischenadapter (5b) zusammengesetzten Rohrstücken (5a) besteht, wobei die Adapter (5b) Düsen (7) zum Zerstäuben der Nährlösung enthalten und die Rohrstücke (5a) 3 - 8 radial abstehende Gitterplatten (18) als Trägerplatten (6) für die Zellen tragen, wobei die Länge des zusammengefügten Zuführungsrohres (5) der Länge des Plastikschlauchs (2) des Fermentergefäßes (1) und der Querschnitt durch die Trägergitter (18) der Öffnung des Plastikschlauches (2) bzw. der Größe des Deckels (3) entspricht.

6. Vorrichtung gemäß Anspruch 5, **dadurch gekennzeichnet, daß** im Außenbereich zwischen den Trägerplatten (6) ein oder mehrere Absaugrohre (8/10) für verbrauchte Nährflüssigkeit und verbrauchte Gase angeordnet sind, welche ebenfalls aus Rohrstücken (8a) bestehen, die den Rohrstücken (5a) des Zuführungsrohrs (5) in der Länge entsprechen und Adapterstücke (8b) zum Verbinden der Rohrstücke (8a) enthalten, sowie einer Absaugdüse am Ende, wobei die Länge dieses Rohres (8) der Länge des Plastikschlauches (2) entspricht.

7. Vorrichtung gemäß Anspruch 1, **dadurch gekennzeichnet, daß** die Trägerplatten (18) quer zur Längsachse des Reaktors angeordnet sind.

8. Vorrichtung gemäß Anspruch 7, **dadurch gekennzeichnet, daß** die Trägerplatten (18) an einer zentralen Halterung (19) über Kippvorrichtungen in einem Winkel von 0-90° zur Halterung eingestellt werden können.

9. Vorrichtung gemäß einem der Ansprüche 1 - 8, **dadurch gekennzeichnet, daß** Deckel (3) und Plastikschlauch (2) über Dichtungen und Spannverbindungen (4) verbunden sind.

10. Vorrichtung gemäß einem der Ansprüche 1 - 8, **dadurch gekennzeichnet, daß** der Deckelteil (3) über eine Schraub- oder Flanschverbindung mit dem Spannverschluß (4) für die Befestigung des Plastikschlauchs (2) verbunden ist.

11. Vorrichtung gemäß einem der Ansprüche 1 - 10, **dadurch gekennzeichnet, daß** eine oder mehrere solcher Vorrichtungen (1) über entsprechende Rohrverbindungen (5, 8) und Haltevorrichtungen (14) an ein zentrales Versorgungssystem (13) angeschlossen sind.

12. Vorrichtung gemäß einem der Ansprüche 1 - 11, **dadurch gekennzeichnet, daß** der Deckelteil (3) aus einem äußeren Deckelring (3a) und einem mittleren Deckel (3b) besteht, wobei alle Nährlösungs- und Gas-Zu- und - abführungsleitungen (5, 8, 9, 10) in dem Ring (3a) vorgesehen sind und an dem mittleren Deckel (3b) über eine Halterung (11) die Trägerplatten (6) befestigt sind und die Zu- und Abführungsleitungen im peripheren Bereich des Fermenters (1) nach unten geführt sind und die Spannverbindung (4) den Plastikschlauch (2) gegen den Deckelring (3a) festhält.

13. Vorrichtung nach Anspruch 12, **dadurch gekennzeichnet, daß** alle Zu- und Abführungsleitungen (5, 8, 9, 10) auf einer Seite des Deckelrings (3a) zugeführt werden und über Ringleitungen (5c, 8c, 9c, 10c) im Deckelring (3a) über den Umfang geleitet werden.

14. Vorrichtung gemäß einem der Ansprüche 1 - 13, **dadurch gekennzeichnet, daß** die Nährlösung über einen Nährlösungstank (13) in Form eines Plastiksacks zirkuliert wird.

## Claims

1. Device for the culturing of plant and animal tissue, comprising a fermenter vessel (1), inlet for liquid nutrients and gases, as well as devices for removal of spent nutrient liquids and gases (8) and solid carrier plates (6) for the tissue which are permeable for the said liquid nutrients and gases and are fixedly arranged within the fermenter vessel (1), **characterised in that** the fermenter vessel (1) consists of a form-stable lid part (3) which carries the various inlet and outlet pipes (5, 8, 9, 10), as well as the carrier plates (6) for the cells and consiste of a cup- or bag-shaped plastic tube (2), the opening of which is sealed off and fixed against the lid (3).

2. Device according to claim 1, **characterised in that** 3 - 8 carrier plates (6) are radially fixed on a central holding means (11) and the supply of the nutrient solution takes place via 3 - 8 supply pipes (5) present in the outer region between the plates (6).

3. Device according to claim 1, **characterised in that** the supply pipes (5a) contain as nozzles bores (7) for the outlet of the nutrient solution and gas inlet pipes (9) are guided parallel to the pipe (5a) which, in appropriate places, also contain bores (7) from which gas emerges.

4. Device according to anyone of claims 1-3, **characterised in that** in the outer region between the carrier plates (6) are arranged one or more sucking-off pipes (8/10) for spent nutrient liquid and spent gas which on the end contain a sucking-off nozzle.

5. Device according to claim 1, **characterised in that** the inlet pipe represents a central pipe (5) which consists of two or more tube pieces (5a) combined via intermediate adapters (5b), whereby the adapters (5b) contain nozzles (7) for the atomisation of the nutrient solution and the tube pieces (5a) carry 3 - 8 radially projecting lattice plates (18) as carrier plates (6) for the cells, whereby the length of the assembled supply pipe (5) corresponds to the length of the plastic tube (2) of the fermenter vessel (1) and the cross-section through the carrier lattice (18) to the opening of the plastic tube (2) or to the size of the lid (3).

6. Device according to claim 5, **characterised in that**, in the outer region between the carrier plates (6), one or more sucking-off pipes (8/10) for spent nutrient liquid and spent gases are arranged which also consist of tube pieces (8a)which correspond in length to the tube pieces (5a) of the supply pipe (5) and contain adapter pieces (8b) for the connecting of the tube pieces (8a), as well as a sucking-off nozzle on the end, whereby the length of this pipe (8) corresponds to the length of the plastic pipe (2).

7. Device according to claim 1, **characterised in that** the carrier plates (18) are arranged transversely to the longitudinal axis of the reactor.

8. Device according to claim 7, **characterised in that** the carrier plates (18) can be adjusted on a central holding means (19) via tilt devices at an angle of 0 - 90°to the holding means.

9. Device according to anyone of claims 1 - 8 **characterised in that** lid (3) and plastic pipe (2) are connected via seals and tension connections (4).

10. Device according to one of claims 1 - 8, **characterised in that** the lid part (3) is connected via a screw or flange connection with the tension closure (4) for the fixing of the plastic pipe (2).

11. Device according to one of claims 1 - 10, **characterised in that** one or more such devices (1) are connected via appropriate pipe connections (5, 8) and holding devices (14) to a central supply system (13).

12. Device according to one of claims 1 - 11, **characterised in that** the lid part (3) consists of an outer lid ring (3a) end a middle lid (3b), whereby all nutrient solution and gas supply and removal pipes (5, 8, 9, 10) are provided in the ring (3a) and on the middle lid (3b) are fixed the carrier plates (6) via holding means (11) and the supply and removal pipes are guided downwardly in the peripheral region of the fermenter (1) and the tension connection (4) firmly holds the plastic pipe (2) against the lid ring (3a).

13. Device according to claim 12, **characterised in that** all supply and removal pipes (5, 8, 9, 10) are provided on one side of the lid ring (3a) and are guided over ring pipes (5c, 8c, 9c, 10c) in the lid ring (3a) over the circumference.

14. Device according to one of claims 1 - 13, **characterised in that** the nutrient solution is circulated via a nutrient solution tank (13) in the form of a plastic sack.

## Revendications

1. Dispositif de culture de tissus végétaux ou animaux, comprenant un récipient fermenteur (1), une alimentation pour les substances nutritives liquides et les gaz (5) ainsi que des dispositifs d'évacuation des liquides nutritifs usés et des gaz usés (8) et des plaques porteuses fixes (6) pour les tissus, qui sont perméables aux dites substances nutritives liquides et aux gaz et sont placées de manière stationnaire à l'intérieur du récipient fermenteur (1), **caractérisé en ce que** le récipient fermenteur (1) est constitué d'une pièce en couvercle (3) indéformable, laquelle porte les différentes conduites d'alimentation et d'évacuation (5, 8, 9, 10) ainsi que les plaques porteuses (6) pour les cellules, et d'un tuyau plastique (2) en forme de pot ou de sac, dont l'ouverture est rendue étanche et est fixée contre le couvercle (3).

2. Dispositif selon la revendication 1, **caractérisé en ce que** 3 à 8 plaques porteuses (6) sont fixées radialement sur une attache centrale (11) et **en ce que** l'amenée de la solution nutritive est réalisée au moyen de 3 à 8 conduites d'alimentation (5) se trouvant dans la partie extérieure entre les plaques (6).

3. Dispositif selon la revendication 1, **caractérisé en ce que** les conduites d'alimentation (5a) comprennent des trous (7), en guise de buses, pour la sortie de la solution nutritive et **en ce que** des conduites d'arrivée de gaz (9) sont menées parallèlement à la conduite (5a), lesquelles, à des emplacements correspondants, comprennent également des trous (7), desquels sort du gaz.

4. Dispositif selon l'une des revendications 1 à 3, **caractérisé en ce que,** dans la partie extérieure entre les plaques porteuses (6), un ou plusieurs tubes d'aspiration (8/10) sont placés pour le liquide nutritif usé et les gaz usés, lesquels comprennent une buse d'aspiration en bout.

5. Dispositif selon la revendication 1, **caractérisé en ce que** la conduite d'alimentation représente un tube central (5), lequel est constitué de deux ou de plusieurs pièces en tube (5a) assemblées par l'intermédiaire d'adaptateurs intermédiaires (5b), les adaptateurs (5b) contenant des buses (7) pour la pulvérisation de la solution nutritive et les pièces en tube (5a) portant, en guise de plaques porteuses (6) des cellules, 3 à 8 plaques en grillage (18) dépassant radialement, la longueur du tube d'alimentation (5) assemblé correspondant à la longueur du tuyau plastique (2) du récipient fermenteur (1) et à la section des grilles porteuses (18) de l'ouverture du tuyau plastique (2) resp. à la taille du couvercle (3).

6. Dispositif selon la revendication 5, **caractérisé en ce que**, dans la partie extérieure entre les plaques porteuses (6), sont placés un ou plusieurs tubes d'aspiration (8/10) pour le liquide nutritif usé et les gaz usés, lesquels sont également constitués de pièces en tubes (8a) dont la longueur correspond aux pièces en tubes (5a) du tube d'alimentation (5) et qui comprennent des adaptateurs (8b) pour raccorder les pièces en tube (8a), ainsi que d'une buse d'aspiration en bout, la longueur de ce tube (8) correspondant à la longueur du tuyau plastique (2).

7. Dispositif selon la revendication 1, **caractérisé en ce que** les plaques porteuses (18) sont placées transversalement à l'axe longitudinal du réacteur.

8. Dispositif selon la revendication 7, **caractérisé en ce que** les plaques porteuses (18) peuvent être ajustées sur une attache centrale (19) dans un angle de 0 à 90° par rapport à l'attache au moyen de dispositifs basculants.

9. Dispositif selon l'une des revendications 1 à 8, **caractérisé en ce que** le couvercle (3) et le tuyau plastique (2) sont raccordés au moyen de joints et de dispositifs tendeurs (4).

10. Dispositif selon l'une des revendications 1 à 8, **caractérisé en ce que** la pièce en couvercle (3) est raccordée à la fermeture de serrage (4) au moyen d'un raccord à vis ou à bride pour la fixation du tuyau plastique (2).

11. Dispositif selon l'une des revendications 1 à 10, **caractérisé en ce qu'**un ou plusieurs de ces dispositifs (1) sont raccordés à un système d'alimentation central (13) par l'intermédiaire de raccords en tube (5, 8) et de dispositifs de maintien (14) correspondants.

12. Dispositif selon l'une des revendications 1 à 11, **caractérisé en ce que** la pièce en couvercle (3) est constituée d'un anneau de couvercle extérieur (3a) et d'un couvercle intermédiaire (3b), toutes les conduites d'alimentation et d'évacuation de la solution nutritive et des gaz (5, 8, 9, 10) étant prévues dans l'anneau (3a) et les plaques porteuses (6) étant fixées sur le couvercle intermédiaire (3b) au moyen d'une attache (11), et les conduites d'alimentation et d'évacuation étant menées vers le bas dans la partie périphérique du fermenteur (1) et le dispositif tendeur (4) maintenant le tuyau plastique (2) contre l'anneau de couvercle (3a).

13. Dispositif selon la revendication 12, **caractérisé en ce que** toutes les conduites d'alimentation et d'évacuation (5, 8, 9, 10) sont amenées d'un côté de l'anneau de couvercle (3a) et sont menées sur le pourtour au moyen de conduites circulaires (5c, 8c, 9c, 10c) dans l'anneau de couvercle (3a).

14. Dispositif selon l'une des revendications 1 à 13, **caractérisé en ce que** la solution nutritive est mise en circulation par l'intermédiaire d'un réservoir de solution nutritive (13) en forme d'un sac en plastique.
